# EUROPEAN PATENT APPLICATION

(11) **EP 3 805 807 A1**
(43) Date of publication of application: **14.04.2021**
(21) Application number: 19815564.0
(22) Date of filing: 30.05.2019
(51) Int. Cl.: G01T 1/161, A61B 5/055, A61B 6/03, A61B 10/00, G06T 7/00

(54) **TOMOGRAPHIC IMAGE PREDICTION DEVICE AND TOMOGRAPHIC IMAGE PREDICTION METHOD**

(30) Priority: 04.06.2018 JP 2018106908
(71) Applicant: Hamamatsu Photonics K.K., Hamamatsu-shi, Shizuoka 435-8558 (JP)
(72) Inventor: KOMORI, Seisaku, Hamamatsu-shi, Shizuoka 435-8558 (JP); KIMURA, Yuichi, Hamamatsu-shi, Shizuoka 435-8558 (JP)
(74) Representative: Grünecker Patent- und Rechtsanwälte PartG mbB
(86) International application number: PCT/JP2019/021637
(87) International publication number: WO 2019/235367

(57) **Abstract**

A tomographic image prediction apparatus 1 includes an input unit 11, a prediction unit 12, an output unit 13, and a learning unit 14. The tomographic image prediction apparatus 1 inputs a tomographic image of a brain of a subject as an input image I1, and predicts a tomographic image of the brain of the subject after the acquisition of the input tomographic image by a deep neural network in the prediction unit 12, and outputs the predicted tomographic image as an output image 12. The tomographic image prediction apparatus 1 is capable of training the deep neural network using a tomographic image database 15. Thus, a tomographic image prediction apparatus and a tomographic image prediction method which are useful for planning a future treatment policy and the like for a subject are realized.

## Description

### Technical Field

The present disclosure relates to an apparatus and a method for predicting a tomographic image.

### Background Art

As the aging of population progresses and the number of dementia patients is increasing, a technique for diagnosing the degree of dementia of a subject is required. The degree of dementia of a subject can be diagnosed by a doctor seeing a tomographic image of a brain as well as from a behavior of the subject. Patent Document 1 discloses a technique for diagnosing a condition of a brain of a subject from a tomographic image of the brain of the subject using a neural network.

### Citation List

### Patent Literature

Patent Document 1: International Publication No. 2008/056638

### Summary of Invention

### Technical Problem

The technique disclosed in Patent Document 1 diagnoses the present degree of dementia from the tomographic image of the present brain of the subject. When the present degree of dementia of the subject can be diagnosed, this is useful for nursing care or treatment for the subject. However, only knowing the present degree of dementia of the subject is not sufficient to develop a policy on nursing care, treatment, or prevention for the subject from the present to the future.

An object of the present invention is to provide a tomographic image prediction apparatus and a tomographic image prediction method which are useful for planning a future treatment policy and the like for a subject.

### Solution to Problem

An embodiment of the present invention is a tomographic image prediction apparatus. The tomographic image prediction apparatus includes (1) an input unit for inputting a tomographic image of a brain of a subject, (2) a prediction unit for predicting, on the basis of the tomographic image of the brain input to the input unit, a tomographic image of the brain of the subject after the acquisition of the input tomographic image, by a deep neural network, and (3) an output unit for outputting a prediction result by the prediction unit.

An embodiment of the present invention is a tomographic image prediction method. The tomographic image prediction method includes (1) an input step of inputting a tomographic image of a brain of a subject, (2) a prediction step of predicting, on the basis of the tomographic image of the brain input in the input step, a tomographic image of the brain of the subject after the acquisition of the input tomographic image, by a deep neural network, and (3) an output step of outputting a prediction result in the prediction step.

### Advantageous Effects of Invention

According to the embodiments of the present invention, by predicting a tomographic image of a future brain of a subject, a more effective policy can be developed on nursing care, treatment, or prevention for the subject from the present to the future.

### Brief Description of Drawings

[FIG. 1] FIG. 1 is a diagram illustrating a configuration of a tomographic image prediction apparatus 1.
[FIG. 2] FIG. 2 is a diagram illustrating a configuration of the tomographic image prediction apparatus 1 with a deep neural network after learning.
[FIG. 3] FIG. 3 is a diagram illustrating a configuration of a tomographic image prediction apparatus 1A.
[FIG. 4] FIG. 4 is a diagram illustrating examples of a tomographic image input to an input unit 11 (input image II), output images I2a to I2d output from an output unit 13, and difference images output from the output unit 13.
[FIG. 5] FIG. 5 is a diagram illustrating actual tomographic images and predicted tomographic images in comparison with each other.
[FIG. 6] FIG. 6 is a diagram illustrating tomographic images predicted in the worst scenario and tomographic images predicted in the best scenario in comparison with each other.
[FIG. 7] FIG. 7 is a diagram illustrating a configuration of a tomographic image prediction apparatus 1B.
[FIG. 8] FIG. 8 is a diagram illustrating a configuration of a tomographic image prediction apparatus 1C.

### Description of Embodiments

Hereinafter, embodiments for carrying out the present invention will be described in detail with reference to the accompanying drawings. In the description of the drawings, the same elements will be denoted by the same reference signs, without redundant description. The present invention is not limited to these examples.

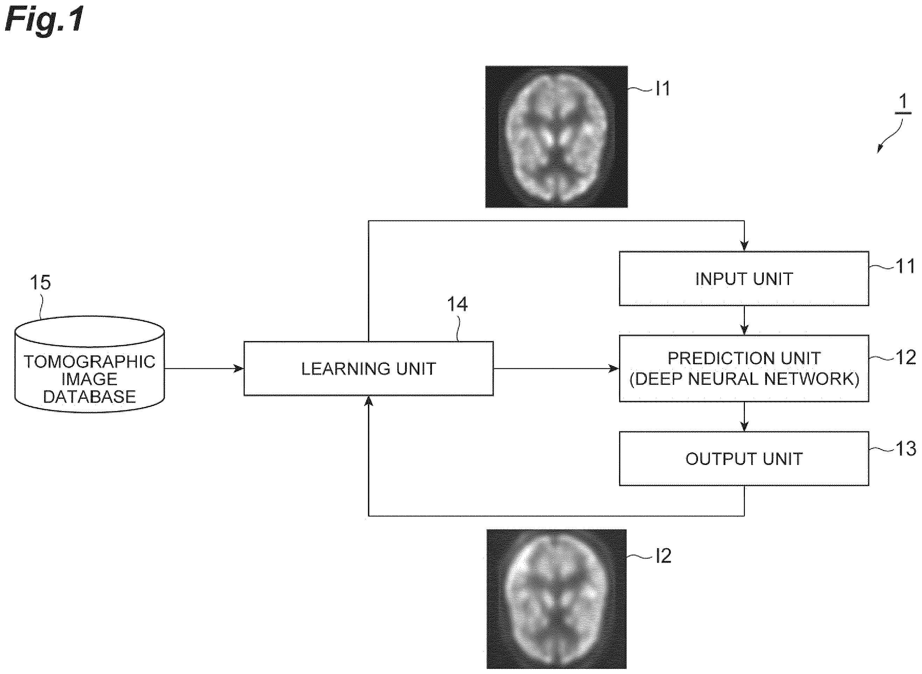

FIG. 1 is a diagram illustrating a configuration of a tomographic image prediction apparatus 1. The tomographic image prediction apparatus 1 inputs a tomographic image of a brain of a subject as an input image I1, predicts a tomographic image of the brain of the subject after the acquisition of the input tomographic image, by a deep neural network, and outputs the predicted tomographic image as an output image 12. Further, the tomographic image prediction apparatus 1 is capable of training the deep neural network using a tomographic image database 15. The tomographic image prediction apparatus 1 is configured using, for example, a computer, and includes an input unit 11, a prediction unit (processing unit) 12, an output unit 13, and a learning unit 14.

The input unit 11 inputs the tomographic image of the brain of the subject as the input image I1. The tomographic image may be any of, for example, a positron emission tomography (PET) image acquired by a PET apparatus, a single photon emission computed tomography (SPECT) image acquired by a SPECT apparatus, and a magnetic resonance imaging (MRI) image acquired by an MRI apparatus. Further, the tomographic image may be a two-dimensional tomographic image of one slice or a plurality of slices, or may be a three-dimensional tomographic image.

The prediction unit 12 predicts, on the basis of the tomographic image of the brain input to the input unit 11 (input image II), a tomographic image of the brain and the like of the subject after the acquisition of the input tomographic image, by the deep neural network. Further, the prediction unit 12 may also predict a health condition of the brain of the subject on the basis of the predicted tomographic image of the brain of the subject. The health condition of the brain is, for example, the degree of dementia, and can be represented by a quantified Z score, or can be represented by a risk level (detailed examination needed, caution needed, follow-up needed, and the like).

The deep neural network may be a convolutional neural network. In the convolutional neural network, a convolutional layer for extracting a feature and a pooling layer for compressing the feature are provided alternately. The prediction unit 12 may perform processing in the deep neural network by a central processing unit (CPU), and further, preferably performs the processing by a digital signal processor (DSP) or a graphics processing unit (GPU) capable of performing faster processing.

The output unit 13 outputs a result of the prediction by the prediction unit 12. The output unit 13 outputs the tomographic image of the brain of the subject predicted by the prediction unit 12 as the output image 12. Further, in a case where the prediction unit 12 also predicts the health condition of the brain of the subject, the output unit 13 also outputs the health condition of the brain of the subject predicted by the prediction unit 12. The output unit 13 may include a display that displays an image.

The learning unit 14 trains the deep neural network of the prediction unit 12 using the tomographic image database 15. The tomographic image database 15 stores data of tomographic images of brains acquired respectively at a plurality of time points for a plurality of subjects. The tomographic image database 15 stores, for example, data of tomographic images of the brain of each subject acquired at substantially one year intervals. The learning unit 14 sets the tomographic image of the subject acquired in a certain year as the input image I1, and causes the deep neural network in the prediction unit 12 to learn on the basis of the output image 12 for the input image I1 and the tomographic image of the subject acquired in the next year (or a year after a lapse of a certain number of years). Such learning of the deep neural network is called deep learning.

A tomographic image prediction method using the above tomographic image prediction apparatus 1 includes an input step by the input unit 11, a prediction step by the prediction unit 12, an output step by the output unit 13, and a learning step by the learning unit 14.

That is, in the input step, a tomographic image of a brain of a subject is input as an input image I1. In the prediction step, on the basis of the tomographic image of the brain input in the input step (input image II), a tomographic image of the brain and the like of the subject after the acquisition of the input tomographic image is predicted by the deep neural network. Further, the prediction step may predict a health condition of the brain of the subject on the basis of the predicted tomographic image of the brain of the subject. In the output step, the tomographic image of the brain of the subject predicted in the prediction step is output as an output image 12, and further, the health condition of the brain of the subject predicted in the prediction step is also output. In the learning step, the deep neural network is trained using the tomographic image database 15.

Once the deep neural network is trained in the learning step, a series of steps of input, prediction and output can be repeatedly performed thereafter, and therefore, there is no need to perform the learning step every time the series of steps of input, prediction and output is performed. For a similar reason, the learning unit 14 is not necessary when the deep neural network is after learning. However, even when the deep neural network is after learning, in a case where further learning is to be performed in order to enable a more accurate prediction, the learning step and the learning unit 14 may be present.

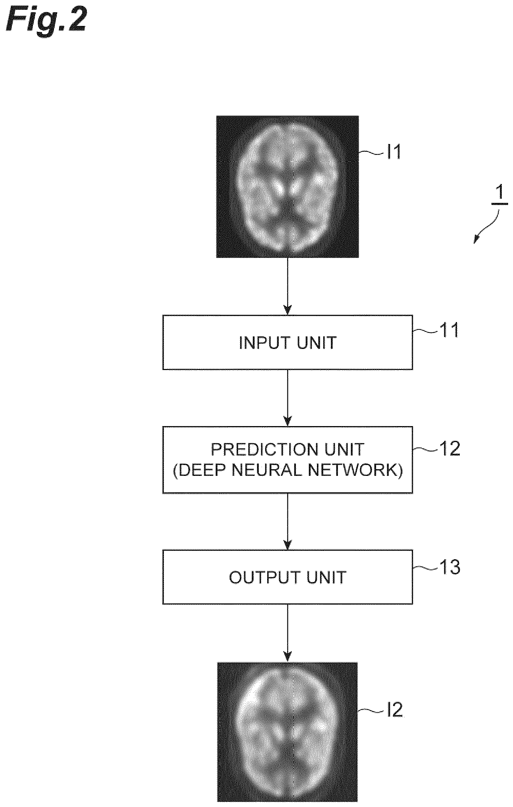

FIG. 2 is a diagram illustrating a configuration of the tomographic image prediction apparatus 1 with the deep neural network after the learning. The tomographic image prediction apparatus 1 is capable of inputting a tomographic image of a brain of a subject as an input image I1, predicting and outputting, as an output image 12, a tomographic image of the brain of the subject after the acquisition of the input tomographic image (for example, one year later, after a lapse of a certain number of years, or the like), by the deep neural network, and further, predicting a health condition of the brain of the subject on the basis of the predicted tomographic image of the brain of the subject. Since a tomographic image of the future brain (and a health condition of the brain) of the subject can be predicted, a more effective policy can be developed on nursing care, treatment, or prevention for the subject from the present to the future.

By predicting the tomographic image of the future brain (and the health condition of the brain) of the subject, for example, the following processes can be taken. In a case where it is determined that there is suspicion of normal pressure hydrocephalus, brain tumor, chronic subdural hematoma, or the like, from the predicted tomographic image, a symptom can be improved by brain surgery. Further, in a case where it is determined that the cause is thyroid hormone abnormality, a symptom can be improved by medical treatment. In a case where it is determined that an Alzheimer type is suspected from the predicted tomographic image, the progress of a symptom can be delayed by administering a drug. Early detection can deal with troubles such as life-related disorders that may occur in the future in advance, and can early determine policies on future treatment, terminal care, nursing care, and the like.

The tomographic image prediction apparatus 1 and the tomographic image prediction method are not limited to the configuration in which one input image I1 is input and one output image 12 is output, and various configurations are possible. Hereinafter, other configurations of the tomographic image prediction apparatus will be described.

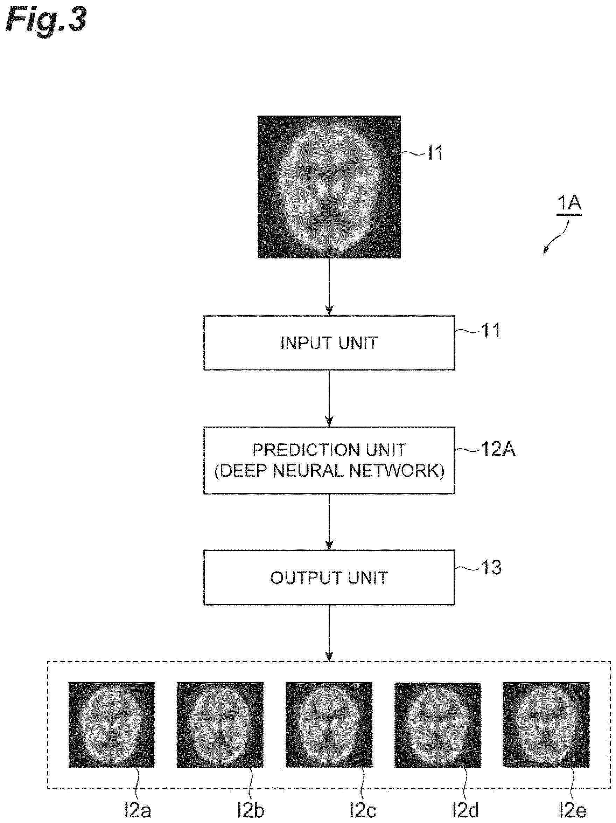

FIG. 3 is a diagram illustrating a configuration of a tomographic image prediction apparatus 1A. In the tomographic image prediction apparatus 1A, a prediction unit 12A predicts tomographic images of a brain respectively at a plurality of time points after the acquisition of the tomographic image of the brain input to the input unit 11 (input image II). The output unit 13 may individually display tomographic images (output images I2a to I2e) of the brain respectively at the plurality of time points predicted by the prediction unit 12A, or may preferably display, as a moving image, the tomographic images of the brain respectively at the plurality of time points by sequentially displaying the images.

For example, the output image I2a is a predicted image after a lapse of one year from the acquisition of the input image I1. The output image I2b is a predicted image after a lapse of two years from the acquisition of the input image I1. The output image I2c is a predicted image after a lapse of three years from the acquisition of the input image I1. The output image I2d is a predicted image after a lapse of four years from the acquisition of the input image I1. Further, the output image I2e is a predicted image after a lapse of five years from the acquisition of the input image I1. In this way, by predicting the tomographic images of the brain respectively at the plurality of future time points, it becomes easy to understand a change in the health condition of the brain.

The prediction unit 12A may predict the output image I2a from the input image I1, predict the output image I2b from the output image I2a, predict the output image I2c from the output image I2b, predict the output image I2d from the output image I2c, and predict the output image I2e from the output image I2d. Further, the deep neural network may be trained so that the output images I2a to I2e can be predicted from the input image I1.

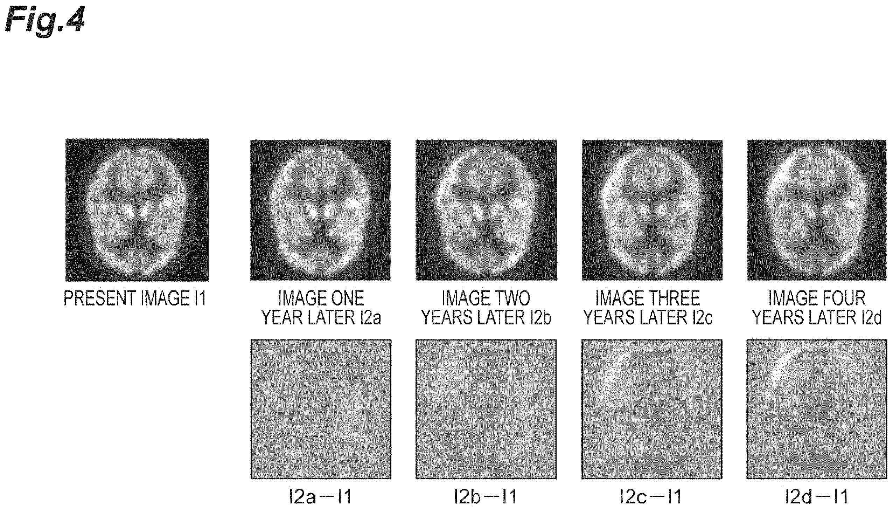

FIG. 4 is a diagram illustrating examples of the tomographic image input to the input unit 11 (input image II), the output images I2a to I2d output from the output unit 13, and difference images output from the output unit 13. As illustrated in this figure, the output unit 13 may obtain the difference images (I2a-I1, I2b-I1, I2c-I1, I2d-I1, and the like) each showing a difference between the tomographic image of the brain predicted by the prediction unit 12A (output images I2a to I2d, or the like) and the tomographic image of the brain input to the input unit 11 (input image I1) and output the difference images. In this way, by displaying the difference images of the brain respectively at the plurality of future time points, it becomes further easier to understand the change in the health condition of the brain (for example, to understand a place and condition to deteriorate).

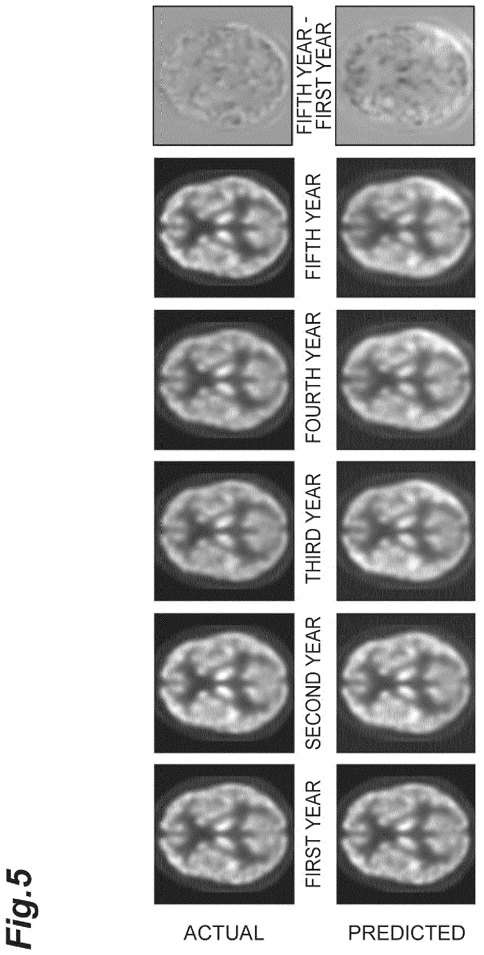

FIG. 5 is a diagram illustrating actual tomographic images and predicted tomographic images in comparison with each other. In this case, the deep neural network was trained using a tomographic image database that stores 470 sets of PET image data, with a PET image at a certain time point and a PET image one year later being as one set. This figure illustrates actual PET images of the first to fifth years of a brain of a specific subject and also illustrates predicted PET images of the first to fifth years of the brain of the specific subject. Further, this figure illustrates a difference image showing a difference between the actual PET image of the fifth year and the PET image of the first year and also illustrates a difference image showing a difference between the predicted PET image of the fifth year and the PET image of the first year.

A comparison between the actual and the prediction shows that there was a common region of decreased activity between the actual PET image of a certain year and the predicted PET image of the same year. Further, the region of decreased activity differs depending on the subject. This indicates that a tomographic image of the future brain can be predicted for each subject from a tomographic image of the brain at a certain time point. Such a result is difficult to obtain by conventional statistical processing.

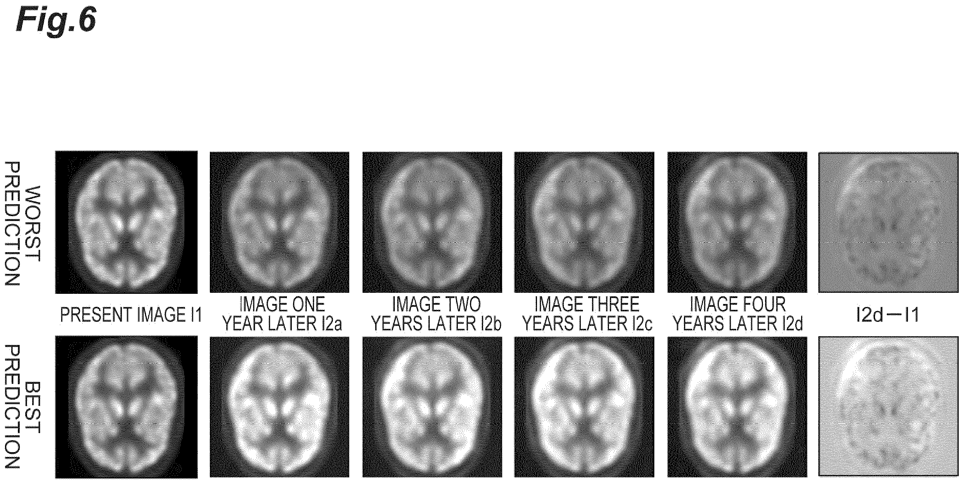

FIG. 6 is a diagram illustrating tomographic images predicted in the worst scenario and tomographic images predicted in the best scenario in companion with each other. As illustrated in this figure, the prediction unit 12A may predict tomographic images of the brain after the acquisition of the tomographic image of the brain input to the input unit 11 respectively for the worst scenario and the best scenario. At this time, the deep neural network is trained using a database of tomographic images different from each other in temporal change rate in the tomographic image database.

When the deep neural network is trained using a database of tomographic images with a fast temporal change (for example, tomographic images of a subject who has a symptom close to Alzheimer-type dementia), a tomographic image of the future brain can be predicted in the worst scenario. When the deep neural network is trained using a database of tomographic images with a slow temporal change (for example, tomographic images of a healthy subject), a tomographic image of the future brain can be predicted in the best scenario. In this way, by predicting the tomographic images of the future brain of the subject respectively in the worst scenario and the best scenario, various policies can be developed on nursing care, treatment, or prevention for the subject from the present to the future.

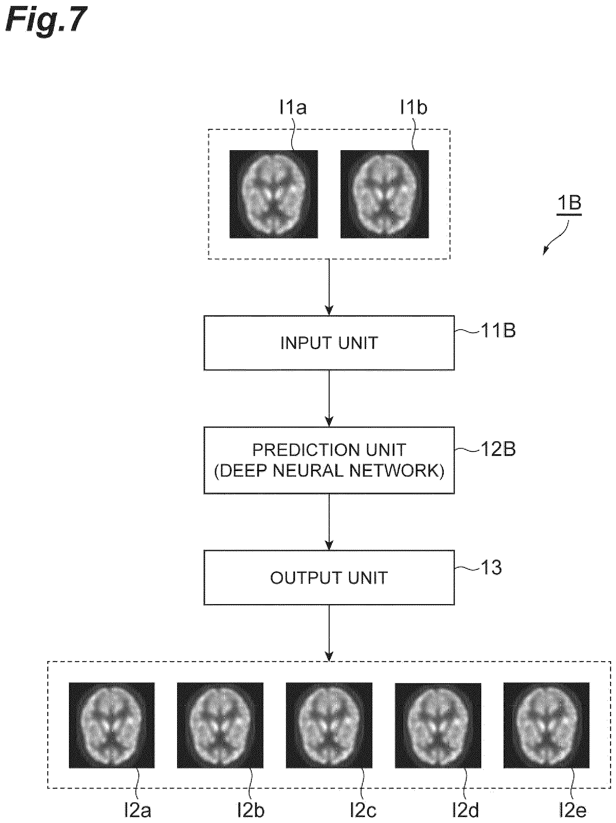

FIG. 7 is a diagram illustrating a configuration of a tomographic image prediction apparatus 1B. In the tomographic image prediction apparatus 1B, an input unit 11B inputs a plurality of tomographic images (input images I1a and I1b) of a brain of a subject. On the basis of the plurality of tomographic images of the brain of the subject input to the input unit 11B, a prediction unit 12B predicts a tomographic image of the brain after the acquisition of those input tomographic images. A deep neural network is trained so that output images I2a to I2e can be predicted from the plurality of tomographic images (input images I1a and I1b) of the brain of the subject.

The plurality of tomographic images of the brain of the subject are tomographic images of the brain of the subject acquired respectively at a plurality of time points (for example, a tomographic image acquired in a certain year and a tomographic image acquired in the next year). Further, the plurality of tomographic images of the brain of the subject are tomographic images of the brain of the subject (for example, a PET image, SPECT image, and MRI image) acquired respectively by a plurality of types of tomographic image acquisition apparatuses. In this way, by predicting a tomographic image of the future brain on the basis of the plurality of tomographic images of the brain of the subject, prediction accuracy can be improved.

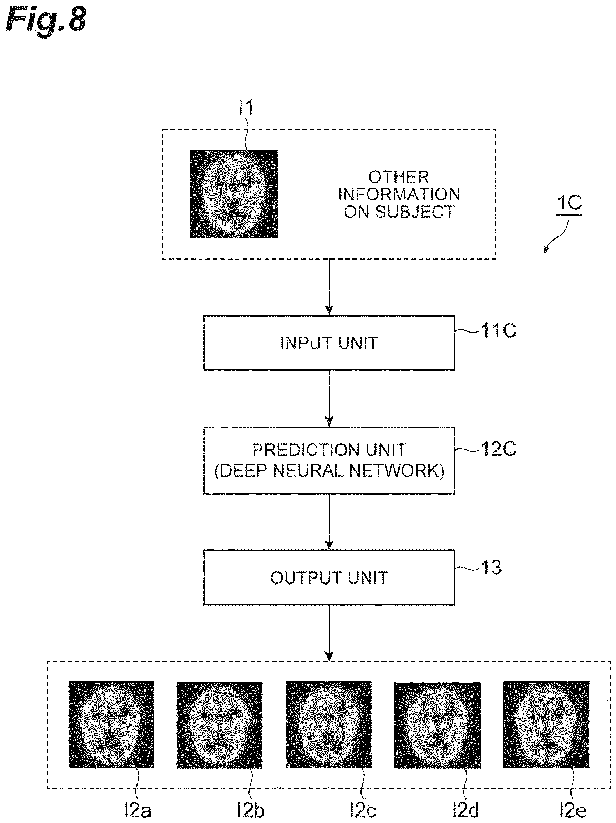

FIG. 8 is a diagram illustrating a configuration of a tomographic image prediction apparatus 1C. In the tomographic image prediction apparatus 1C, an input unit 11C not only inputs a tomographic image of a brain of a subject (input image I1) but also inputs other information on the subject. On the basis of the tomographic image of the brain of the subject input to the input unit 11C and other information on the subject, a prediction unit 12C predicts a tomographic image of the brain after the acquisition of the input tomographic image. A deep neural network is trained so that output images I2a to I2e can be predicted from the tomographic image of the brain of the subject and other information on the subject.

The other information on the subject is information that may be related to a health condition of the brain, and is, for example, information such as age, sex, genetic information, medical history, and lifestyle habit. In this way, by predicting a tomographic image of the future brain on the basis of the tomographic image of the brain of the subject and other information on the subject, prediction accuracy can be improved.

The present invention is not limited to the above embodiments and configuration examples, and various modifications can be made. For example, any of the various configurations described above may be combined.

A medical institution equipped with the tomographic image acquisition apparatus (PET apparatus, SPECT apparatus, MRI apparatus) and for acquiring a tomographic image of a brain of a subject and an institution equipped with the tomographic image prediction apparatus (hereinafter referred to as "prediction institution") and for predicting a tomographic image of a future brain of a subject may be separate.

In this case, the tomographic image of the brain of the subject acquired in the medical institution is transmitted to the prediction institution through a communication line between the medical institution and the prediction institution, and the tomographic image of the future brain predicted in the prediction institution is transmitted to the medical institution. Then, a doctor or the like in the medical institution can develop an effective policy on nursing care, treatment or prevention for the subject from the present to the future, on the basis of the tomographic image of the future brain transmitted from the prediction institution.

The tomographic image prediction apparatus of the above embodiment includes (1) an input unit for inputting a tomographic image of a brain of a subject, (2) a prediction unit for predicting, on the basis of the tomographic image of the brain input to the input unit, a tomographic image of the brain of the subject after the acquisition of the input tomographic image, by a deep neural network, and (3) an output unit for outputting a prediction result by the prediction unit.

The above tomographic image prediction apparatus may further include a learning unit for training the deep neural network using a database of tomographic images of brains acquired respectively at a plurality of time points for a plurality of subjects.

In the above tomographic image prediction apparatus, the prediction unit may predict, on the basis of the predicted tomographic image of the brain of the subject, a health condition of the brain of the subject.

In the above tomographic image prediction apparatus, the prediction unit may predict tomographic images of the brain respectively at a plurality of time points after the acquisition of the tomographic image of the brain input to the input unit. Further, in this case, the output unit may display, as a moving image, the tomographic images of the brain respectively at the plurality of time points predicted by the prediction unit.

In the above tomographic image prediction apparatus, the output unit may obtain a difference image showing a difference between the tomographic image of the brain predicted by the prediction unit and the tomographic image of the brain input to the input unit, and output the difference image.

In the above tomographic image prediction apparatus, the prediction unit may predict the tomographic image of the brain after the acquisition of the tomographic image of the brain input to the input unit, by the deep neural network being trained using a database of tomographic images different from each other in temporal change rate in a database of tomographic images of brains acquired respectively at a plurality of time points for a plurality of subjects.

In the above tomographic image prediction apparatus, the prediction unit may predict, on the basis of tomographic images of the brain of the subject acquired respectively at a plurality of time points, a tomographic image of the brain after the acquisition of those tomographic images. Further, the prediction unit may predict, on the basis of tomographic images of the brain of the subject acquired respectively by a plurality of types of tomographic image acquisition apparatuses, a tomographic image of the brain after the acquisition of those tomographic images. Further, the prediction unit may predict, on the basis of the tomographic image of the brain input to the input unit and other information on the subject, a tomographic image of the brain after the acquisition of the input tomographic image.

The tomographic image prediction method of the above embodiment includes (1) an input step of inputting a tomographic image of a brain of a subject, (2) a prediction step of predicting, on the basis of the tomographic image of the brain input in the input step, a tomographic image of the brain of the subject after the acquisition of the input tomographic image, by a deep neural network, and (3) an output step of outputting a prediction result in the prediction step.

The above tomographic image prediction method may further include a learning step of training the deep neural network using a database of tomographic images of brains acquired respectively at a plurality of time points for a plurality of subjects.

In the above tomographic image prediction method, the prediction step may predict, on the basis of the predicted tomographic image of the brain of the subject, a health condition of the brain of the subject.

In the above tomographic image prediction method, the prediction step may predict tomographic images of the brain respectively at a plurality of time points after the acquisition of the tomographic image of the brain input in the input step. Further, in this case, the output step may display, as a moving image, the tomographic images of the brain respectively at the plurality of time points predicted in the prediction step.

In the above tomographic image prediction method, the output step may obtain a difference image showing a difference between the tomographic image of the brain predicted in the prediction step and the tomographic image of the brain input in the input step, and output the difference image.

In the above tomographic image prediction method, the prediction step may predict the tomographic image of the brain after the acquisition of the tomographic image of the brain input in the input step, by the deep neural network being trained using a database of tomographic images different from each other in temporal change rate in a database of tomographic images of brains acquired respectively at a plurality of time points for a plurality of subjects.

In the above tomographic image prediction method, the prediction step may predict, on the basis of tomographic images of the brain of the subject acquired respectively at a plurality of time points, a tomographic image of the brain after the acquisition of those tomographic images. Further, the prediction step may predict, on the basis of tomographic images of the brain of the subject acquired respectively by a plurality of types of tomographic image acquisition apparatuses, a tomographic image of the brain after the acquisition of those tomographic images. Further, the prediction step may predict, on the basis of the tomographic image of the brain input in the input step and other information on the subject, a tomographic image of the brain after the acquisition of the input tomographic image.

### Industrial Applicability

The present invention can be used as a tomographic image prediction apparatus and a tomographic image prediction method which are useful for planning a future treatment policy and the like for a subject.

### Reference Signs List

1, 1A - 1C - tomographic image prediction apparatus, 11, 11B, 11C - input unit, 12, 12A - 12C - prediction unit, 13 - output unit, 14 - learning unit, 15 - tomographic image database.

## Claims

1. A tomographic image prediction apparatus comprising:
an input unit for inputting a tomographic image of a brain of a subject;
a prediction unit for predicting, on the basis of the tomographic image of the brain input to the input unit, a tomographic image of the brain of the subject after the acquisition of the input tomographic image, by a deep neural network; and
an output unit for outputting a prediction result by the prediction unit.

2. The tomographic image prediction apparatus according to Claim 1, further comprising a learning unit for training the deep neural network using a database of tomographic images of brains acquired respectively at a plurality of time points for a plurality of subjects.

3. The tomographic image prediction apparatus according to Claim 1 or 2, wherein the prediction unit predicts, on the basis of the predicted tomographic image of the brain of the subject, a health condition of the brain of the subject.

4. The tomographic image prediction apparatus according to any one of Claims 1 to 3, wherein the prediction unit predicts tomographic images of the brain respectively at a plurality of time points after the acquisition of the tomographic image of the brain input to the input unit.

5. The tomographic image prediction apparatus according to Claim 4, wherein the output unit displays, as a moving image, the tomographic images of the brain respectively at the plurality of time points predicted by the prediction unit.

6. The tomographic image prediction apparatus according to any one of Claims 1 to 5, wherein the output unit obtains a difference image showing a difference between the tomographic image of the brain predicted by the prediction unit and the tomographic image of the brain input to the input unit, and outputs the difference image.

7. The tomographic image prediction apparatus according to any one of Claims 1 to 6, wherein the prediction unit predicts the tomographic image of the brain after the acquisition of the tomographic image of the brain input to the input unit, by the deep neural network being trained using a database of tomographic images different from each other in temporal change rate in a database of tomographic images of brains acquired respectively at a plurality of time points for a plurality of subjects.

8. The tomographic image prediction apparatus according to any one of Claims 1 to 7, wherein the prediction unit predicts, on the basis of tomographic images of the brain of the subject acquired respectively at a plurality of time points, a tomographic image of the brain after the acquisition of those tomographic images.

9. The tomographic image prediction apparatus according to any one of Claims 1 to 8, wherein the prediction unit predicts, on the basis of tomographic images of the brain of the subject acquired respectively by a plurality of types of tomographic image acquisition apparatuses, a tomographic image of the brain after the acquisition of those tomographic images.

10. The tomographic image prediction apparatus according to any one of Claims 1 to 9, wherein the prediction unit predicts, on the basis of the tomographic image of the brain input to the input unit and other information on the subject, a tomographic image of the brain after the acquisition of the input tomographic image.

11. A tomographic image prediction method comprising:
an input step of inputting a tomographic image of a brain of a subject;
a prediction step of predicting, on the basis of the tomographic image of the brain input in the input step, a tomographic image of the brain of the subject after the acquisition of the input tomographic image, by a deep neural network; and
an output step of outputting a prediction result in the prediction step.

12. The tomographic image prediction method according to Claim 11, further comprising a learning step of training the deep neural network using a database of tomographic images of brains acquired respectively at a plurality of time points for a plurality of subjects.

13. The tomographic image prediction method according to Claim 11 or 12, wherein the prediction step predicts, on the basis of the predicted tomographic image of the brain of the subject, a health condition of the brain of the subject.

14. The tomographic image prediction method according to any one of Claims 11 to 13, wherein the prediction step predicts tomographic images of the brain respectively at a plurality of time points after the acquisition of the tomographic image of the brain input in the input step.

15. The tomographic image prediction method according to Claim 14, wherein the output step displays, as a moving image, the tomographic images of the brain respectively at the plurality of time points predicted in the prediction step.

16. The tomographic image prediction method according to any one of Claims 11 to 15, wherein the output step obtains a difference image showing a difference between the tomographic image of the brain predicted in the prediction step and the tomographic image of the brain input in the input step, and outputs the difference image.

17. The tomographic image prediction method according to any one of Claims 11 to 16, wherein the prediction step predicts the tomographic image of the brain after the acquisition of the tomographic image of the brain input in the input step, by the deep neural network being trained using a database of tomographic images different from each other in temporal change rate in a database of tomographic images of brains acquired respectively at a plurality of time points for a plurality of subjects.

18. The tomographic image prediction method according to any one of Claims 11 to 17, wherein the prediction step predicts, on the basis of tomographic images of the brain of the subject acquired respectively at a plurality of time points, a tomographic image of the brain after the acquisition of those tomographic images.

19. The tomographic image prediction method according to any one of Claims 11 to 18, wherein the prediction step predicts, on the basis of tomographic images of the brain of the subject acquired respectively by a plurality of types of tomographic image acquisition apparatuses, a tomographic image of the brain after the acquisition of those tomographic images.

20. The tomographic image prediction method according to any one of Claims 11 to 19, wherein the prediction step predicts, on the basis of the tomographic image of the brain input in the input step and other information on the subject, a tomographic image of the brain after the acquisition of the input tomographic image.
